(19) Europäisches Patentamt
European Patent Office
Office européen des brevets

(11) **EP 4 385 724 A1**

(12) **EUROPEAN PATENT APPLICATION**
published in accordance with Art. 153(4) EPC

(43) Date of publication:
**19.06.2024 Bulletin 2024/25**

(21) Application number: **22855847.4**

(22) Date of filing: **04.08.2022**

(51) International Patent Classification (IPC):
*B32B 5/32* (2006.01)   *B01D 69/00* (2006.01)
*B01D 69/02* (2006.01)   *B01D 69/10* (2006.01)
*B01D 69/12* (2006.01)   *B01D 71/14* (2006.01)
*B01D 71/26* (2006.01)   *B32B 27/32* (2006.01)
*C12M 1/00* (2006.01)   *C12N 1/02* (2006.01)

(52) Cooperative Patent Classification (CPC):
**B01D 69/00; B01D 69/02; B01D 69/10;
B01D 69/12; B01D 71/14; B01D 71/26; B32B 5/32;
B32B 27/32; C12M 1/00; C12N 1/02**

(86) International application number:
**PCT/JP2022/030003**

(87) International publication number:
**WO 2023/017785 (16.02.2023 Gazette 2023/07)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.08.2021 JP 2021130802**

(71) Applicant: **Teijin Limited
Osaka 530-0005 (JP)**

(72) Inventor: **IKUTA, Mami
Osaka-shi, Osaka 530-0005 (JP)**

(74) Representative: **Carpmaels & Ransford LLP
One Southampton Row
London WC1B 5HA (GB)**

(54) **LAMINATED FILM**

(57)    A laminated film (10A) has a polyolefin microporous membrane (20) and a porous support layer (30), in which the polyolefin microporous membrane (20) and the porous support layer (30) are bonded to each other by bonding parts (40) that contain a thermoplastic resin and are scattered, and the laminated film has a Gurley value of from 5 sec/100 mL to 100 sec/100 mL.

FIG.1

**Description**

Technical Field

The present disclosure relates to a laminated film.

Background Art

Japanese National-Phase Publication (JP-A) No. 2011-512252 discloses a membrane made of a porous polyethylene that can be used for filtration media.
Japanese Patent Application Laid-Open (JP-A) No. 2015-163465 discloses a porous laminated body, which includes a polyolefin-based porous film and a polyolefin-based fiber layer, and is used for a medical sterile packaging material.
Japanese Patent Application Laid-Open (JP-A) No. H11-179120 discloses a laminated filter made of a polyolefin resin, in which a polyolefin nonwoven fabric and a polyolefin microporous membrane are integrated by thermal calendering, and which is useful as an industrial liquid filtration filter.
Japanese Patent Application Laid-Open (JP-A) No. 2008-114530 discloses a method for producing a composite sheet, in which an olefinic porous film and an air permeable reinforcement are bonded with a hot-melt adhesive.

SUMMARY OF INVENTION

Technical Problem

Conventionally, it has been difficult to achieve both a low pressure loss and bacteria separation performance in a laminated film in which a polyolefin microporous membrane and a porous support layer are layered.
An object of the disclosure is to provide a laminated film having a low initial pressure loss and excellent bacteria separation performance.

Solution to Problem

Specific means for solving the problem include the following aspects.

<1> A laminated film, including a microporous membrane containing a polyolefin, and a porous support layer, in which the microporous membrane and the porous support layer are bonded to each other by bonding parts that contain a thermoplastic resin and are scattered, and the laminated film has a Gurley value of from 5 sec/100 mL to 100 sec/100 mL.
<2> The laminated film according to <1>, in which the microporous membrane contains polyethylene.
<3> The laminated film according to <2>, in which the polyethylene contained in the microporous membrane has a weight average molecular weight of from 800,000 to 2,800,000.
<4> The laminated film according to any one of <1> to <3>, in which the microporous membrane has a Gurley value of from 2 sec/100 mL to 100 sec/100 mL.
<5> The laminated film according to any one of <1> to <4>, in which the microporous membrane has a porosity of from 80% to 90%.
<6> The laminated film according to any one of <1> to <5>, in which the microporous membrane has an average thickness of from 10 $\mu$m to 110 $\mu$m.
<7> The laminated film according to any one of <1> to <6>, in which the porous support layer is a polyester fiber structure.
<8> The laminated film according to any one of <1> to <7>, in which the porous support layer has a basis weight of from 50 g/m$^2$ to 150 g/m$^2$.
<9> The laminated film according to any one of <1> to <8>, in which the porous support layer has a bulk density of from 0.20 g/cm$^3$ to 0.50 g/cm$^3$.
<10> The laminated film according to any one of <1> to <9>, in which the laminated film is used for separating bacteria.

Advantageous Effects of Invention

According to the disclosure, a laminated film having a low initial pressure loss and excellent bacteria separation performance is provided.

BRIEF DESCRIPTION OF DRAWINGS

Fig. 1 is a cross-sectional view showing an example of an embodiment of a laminated film.
Fig. 2 is a cross-sectional view showing another example of an embodiment of a laminated film.

DESCRIPTION OF EMBODIMENTS

Hereinafter, embodiments of the disclosure will be described. These descriptions and examples illustrate embodiments and do not limit the scope of the embodiments.

In the disclosure, a numerical range indicated using "to" indicates a range including numerical values described before and after "to" as a minimum value and a maximum value, respectively.

In the numerical ranges described in stages in the disclosure, the upper limit or the lower limit described in one numerical range may be replaced with the upper limit or the lower limit of a numerical range described in another stage. In addition, in a numerical range described in the disclosure, the upper limit or the lower limit of the numerical range may be replaced with a value shown in an example.

In the disclosure, the term "step" includes not only an independent step but also a step that cannot be clearly distinguished from other steps as long as the intended purpose of the step is achieved.

In a case in which the amount of each component in a composition is referred to in the disclosure, if there are a plurality of substances corresponding to each component in the composition, the amount means the total amount of the plurality of substances present in the composition unless otherwise specified.

In the disclosure, a machine direction (MD) means a longitudinal direction in a membrane and a laminated film produced in an elongated shape, and a transverse direction (TD) means a direction orthogonal to the MD in a plane direction of the membrane and the laminated film. In the disclosure, TD is also referred to as a "width direction".

In the disclosure, with respect to the laminated film, a side into which a gas or a liquid flows is referred to as "upstream", and a side from which a gas or a liquid flows out is referred to as "downstream".

In the disclosure, a microporous membrane containing a polyolefin is referred to as a "polyolefin microporous membrane".

In the disclosure, a "porous support layer" does not include a "polyolefin microporous membrane". The "porous support layer" is a sheet-like object other than a "polyolefin microporous membrane".

In the disclosure, as the melting points of a polyolefin of a polyolefin microporous membrane and a thermoplastic resin included in the laminated film, the melting points of a polyolefin of a polyolefin microporous membrane and a thermoplastic resin, which are materials used for producing the laminated film, may be used, respectively.

<Laminated Film>

The laminated film of the disclosure includes a polyolefin microporous membrane and a porous support layer, in which the polyolefin microporous membrane and the porous support layer are bonded to each other by bonding parts that contain a thermoplastic resin and are scattered, and the laminated film has a Gurley value of from 5 sec/100 mL to 100 sec/100 mL.

In the laminated film of the disclosure, the polyolefin microporous membrane and the porous support layer are bonded to each other by the bonding parts containing a thermoplastic resin. Since the thermoplastic resin is melted by applying heat to bond the polyolefin microporous membrane and the porous support layer to each other, the bonding can be achieved at a lower temperature as compared with a case in which a thermal calendering treatment is performed to directly bond the polyolefin microporous membrane and the porous support layer to each other by heat, and the porous structures of the polyolefin microporous membrane and the porous support layer are less likely to be blocked.

In the laminated film of the disclosure, since the polyolefin microporous membrane and the porous support layer are bonded to each other by bonding parts that are scattered, the laminated film has favorable air permeability and the initial pressure loss can be suppressed.

The laminated film of the disclosure has a Gurley value of 100 sec/100 mL or less. In a laminated film having a Gurley value of more than 100 sec/100 mL, there is a possibility that the pore size is too small or some pores are blocked, and such a laminated film tends to have a large initial pressure loss. The laminated film of the disclosure has a Gurley value of 100 sec/100 mL or less, preferably 70 sec/100 mL or less, more preferably 30 sec/100 mL or less, and still more preferably 20 sec/100 mL or less from the viewpoint of reducing the initial pressure loss.

The laminated film of the disclosure has a Gurley value of 5 sec/100 mL or more. In a laminated film having a Gurley value of less than 5 sec/100 mL, the pore size is too large, and sufficient bacteria separation performance cannot be obtained. The laminated film of the disclosure has a Gurley value of 5 sec/100 mL or more, preferably 6 sec/100 mL or more, and more preferably 7 sec/100 mL or more from the viewpoint of excellent bacteria separation performance.

In the disclosure, the Gurley value of the laminated film is a value measured according to JIS P 8117: 2009.

[Use of Laminated Film]

The laminated film of the disclosure is suitable for use in separating a biological particle by circulating a gas or a liquid, and is particularly suitable for use in separating bacteria among biological particles.

Examples of the biological particle as used herein include a particle possessed by an organism, a particle released by an organism, a particle parasitic on an organism, a minute organism, a vesicle having a lipid as a membrane, and fragments thereof. Examples of the biological particle as used herein include viruses, parts of viruses (for example, a particle obtained by removing an envelope from a virus having an envelope), bacteriophages, bacteria, endospores, spores, fungi, mold, yeast, cysts, protozoa, unicellular algae, plant cells, animal cells, cultured cells, hybridomas, tumor cells, erythrocytes, leukocytes (for example, lymphocytes, monocytes, and granulocytes), platelets, cell organelles (for example, cell nuclei, mitochondria, and vesicles), exosomes, apoptotic bodies, lipid bilayer particles, lipid monolayer particles, liposomes, enzymes, aggregates of enzymes, proteins, aggregates of proteins, and fragments thereof. Examples of the biological particle as used herein also include artifacts.

There is no limit on the size of the biological particle to be separated by the laminated film of the disclosure. The diameter or long axis length of the biological particle is, for example, 1 nm or more, 5 nm or more, 10 nm or more, or 20 nm or more, and for example, 100 $\mu$m or less, 50 $\mu$m or less, 10 $\mu$m or less, or 5 $\mu$m or less.

As the bacteria to be separated by the laminated film of the disclosure, nano-sized or micro-sized bacteria are suitable. In this case, the diameter or long axis length of the particle is preferably 100 nm or more, more preferably 200 nm or more, still more preferably 300 nm or more, preferably 5 $\mu$m or less, more preferably 4 $\mu$m or less, and still more preferably 3 $\mu$m or less.

The laminated film of the disclosure is suitable as a filter medium for an air filter that prevents entry of bacteria. The air filter is, for example, a dustproof mask, a medical mask, a coarse dust air filter, a medium performance air filter, a high performance air filter, or an ultra high performance air filter.

The use of the laminated film of the disclosure is not limited to a filter medium for an air filter. Examples of the use of the laminated film of the disclosure include a bag-shaped body for capturing a functional particle. Examples of the functional particle to be captured include a biological particle, a resin particle, a metal particle, a mineral particle, and a ceramic particle; a pharmaceutical product, a food, an enzyme, a catalyst, a microorganism, a gas absorber, a dehumidifying agent, a deodorant, and a heat generating agent.

The bag-shaped body is produced, for example, by folding or superimposing the laminated films cut into a predetermined shape and dimension, and then partially or entirely bonding the outer peripheral edges of the superimposed laminated films.

[Layer Configuration of Laminated Film]

The laminated film of the disclosure has at least one layer of the polyolefin microporous membrane and at least one layer of the porous support layer. The laminated film of the disclosure may have a plurality of layers of the polyolefin microporous membrane or a plurality of layers of the porous support layer. The laminated film of the disclosure is preferably a laminated film having a layer configuration in which at least one layer of the porous support layer is layered on one surface of one layer of the polyolefin microporous membrane. The laminated film of the disclosure may have another layer different from the polyolefin microporous membrane and the porous support layer.

The layer configuration of the laminated film of the disclosure will be described with reference to the drawings. Components denoted using the same reference numerals in each drawing mean the same or similar components. The sizes of the members in each drawing are conceptual, and the relative size relationship between the members is not limited thereto. The configuration of the laminated film of the disclosure is not limited to the configuration shown in the drawings.

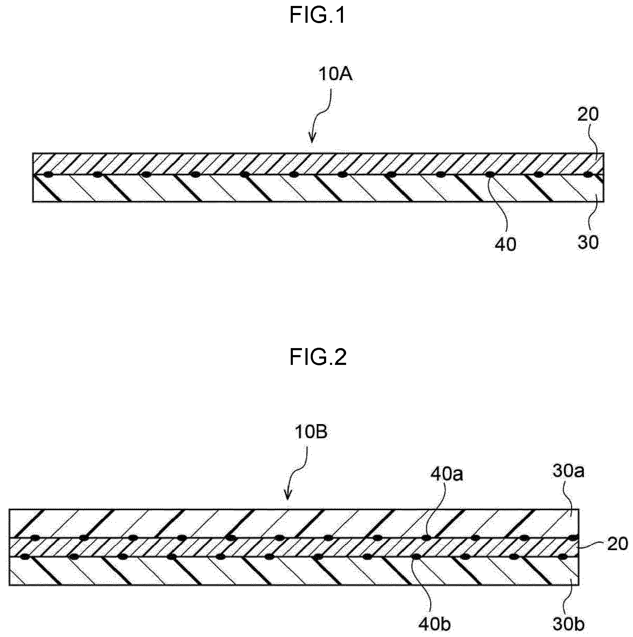

Fig. 1 is a cross-sectional view showing an example of an embodiment of the laminated film. A laminated film 10A shown in Fig. 1 has a polyolefin microporous membrane 20 and a porous support layer 30. The porous support layer 30 is disposed on one surface of the polyolefin microporous membrane 20.

In the laminated film 10A, the polyolefin microporous membrane 20 and the porous support layer 30 are bonded to each other by bonding parts 40. The bonding parts 40 are scattered at the boundary surface between the polyolefin microporous membrane 20 and the porous support layer 30. When the boundary surface is viewed in a plan view, the bonding parts 40 are scattered in the form of, for example, a dot, a line, a lattice, or a net. The bonding part 40 contains a thermoplastic resin. The bonding part 40 preferably contains only a thermoplastic resin.

In the laminated film 10A, it is preferable that the polyolefin microporous membrane 20 is on the upstream side and the porous support layer 30 is on the downstream side during use.

The laminated film 10A may have a reinforcing layer, an adhesive layer, a protective layer, or the like on the peripheral edge of one or both exposed surfaces.

The laminated film 10A is regarded as one unit, and a plurality of these units may be stacked to form a multilayer laminated film, and this multilayer laminated film is an example of an embodiment of the laminated film of the disclosure.

Fig. 2 is a cross-sectional view showing another example of an embodiment of the laminated film. A laminated film 10B shown in Fig. 2 has a polyolefin microporous membrane 20, a porous support layer 30a, and a porous support layer 30b. The porous support layer 30a is disposed on one surface of the polyolefin microporous membrane 20, and the porous support layer 30b is disposed on the other surface of the polyolefin microporous membrane 20. The porous support layer 30a and the porous support layer 30b may be the same type of porous support layer or different types of porous support layers in terms of material, thickness, porosity, basis weight, or the like.

In the laminated film 10B, the polyolefin microporous membrane 20 and the porous support layer 30a are bonded to each other by bonding parts 40a, and the polyolefin microporous membrane 20 and the porous support layer 30b are bonded to each other by bonding parts 40b. The bonding parts 40a are scattered at the boundary surface between the polyolefin microporous membrane 20 and the porous support layer 30a. The bonding parts 40b are scattered at the boundary surface between the polyolefin microporous membrane 20 and the porous support layer 30b. When the boundary surface is viewed in a plan view, the bonding parts 40a and the bonding parts 40b are each scattered in the form of, for example, a dot, a line, a lattice, or a net. The bonding part 40a and the bonding part 40b contain a thermoplastic resin. The bonding part 40a and the bonding part 40b preferably contain only a thermoplastic resin. The bonding part 40a and the bonding part 40b may be the same type of bonding part or different types of bonding parts in terms of the type of thermoplastic resin. The bonding part 40a and the bonding part 40b may be the same type of bonding part or different types of bonding parts in terms of the scattered form.

The laminated film 10B may have a reinforcing layer, an adhesive layer, a protective layer, or the like on the peripheral edge of one or both exposed surfaces.

The laminated film 10B is regarded as one unit, and a plurality of these units may be stacked to form a multilayer laminated film, and this multilayer laminated film is an example of an embodiment of the laminated film of the disclosure.

Hereinafter, details of each layer included in the laminated film of the disclosure will be described.

[Polyolefin Microporous Membrane]

In the disclosure, the microporous membrane means a membrane, which has many micropores inside and has a structure in which micropores are connected, and through which a gas or a liquid can pass from one surface to the other surface.

The polyolefin microporous membrane preferably has a three-dimensional network structure made of polyolefin fibrils.

Examples of the polyolefin contained in the polyolefin microporous membrane include polyethylene, polypropylene, polybutylene, polymethylpentene, and a copolymer of polypropylene and polyethylene. Among these materials, polyethylene is preferable, and high-density polyethylene, a mixture of high-density polyethylene and ultra-high molecular weight polyethylene, and the like are suitable.

An example of an embodiment of the polyolefin microporous membrane includes a polyethylene microporous membrane in which the polyolefin contained is only polyethylene.

An example of an embodiment of the polyolefin microporous membrane includes a microporous membrane containing polypropylene from the viewpoint of having heat resistance so that the membrane does not rupture easily when being exposed to a high temperature.

An example of an embodiment of the polyolefin microporous membrane includes a polyolefin microporous membrane containing at least polyethylene and polypropylene in admixture.

An example of an embodiment of the polyolefin microporous membrane includes a polyolefin microporous membrane, which has a laminated structure of two or more layers, and in which at least one layer contains polyethylene and at least one layer contains polypropylene.

The thickness of the polyolefin microporous membrane is preferably 10 $\mu$m or more, more preferably 12 $\mu$m or more, and still more preferably 14 $\mu$m or more from the viewpoint of increasing the strength of the polyolefin microporous membrane and increasing the separation rate of a biological particle.

The thickness of the polyolefin microporous membrane is preferably 110 $\mu$m or less, more preferably 100 $\mu$m or less, and still more preferably 80 $\mu$m or less from the viewpoint of reducing initial pressure loss of the laminated film.

The thickness of the polyolefin microporous membrane is determined by measurement at 20 points with a contact-type film thickness meter and averaging the measurements.

The porosity of the polyolefin microporous membrane is preferably 80% or more, more preferably 81% or more, and still more preferably 82% or more from the viewpoint of reducing initial pressure loss of the laminated film.

The porosity of the polyolefin microporous membrane is preferably 90% or less, more preferably 88% or less, and still more preferably 86% or less from the viewpoint of increasing the separation rate of a biological particle.

The porosity of the polyolefin microporous membrane is determined by the following calculation method.

That is, regarding a component material 1, a component material 2, a component material 3, ..., and a component material n of the polyolefin microporous membrane, when the mass of each component material is denoted by $W_1$, $W_2$, $W_3$, ..., and $W_n$ (g/cm$^2$), the true density of each component material is denoted by $d_1$, $d_2$, $d_3$, ..., and $d_n$ (g/cm$^3$), and the membrane thickness is denoted by t (cm), the porosity $\varepsilon$ (%) is determined by the following mathematical formula.

$$\varepsilon = \left( 1 - \frac{\sum_{i=1}^{n} \frac{Wi}{di}}{t} \right) \times 100$$

The Gurley value of the polyolefin microporous membrane is preferably 2 sec/100 mL or more, more preferably 3 sec/100 mL or more, and still more preferably 5 sec/100 mL or more from the viewpoint of increasing the separation rate of a biological particle.

The Gurley value of the polyolefin microporous membrane is preferably 100 sec/100 mL or less, more preferably 90 sec/100 mL or less, and still more preferably 70 sec/100 mL or less from the viewpoint of reducing initial pressure loss.

The Gurley value of the polyolefin microporous membrane is a value measured according to JIS P 8117: 2009.

The weight average molecular weight (Mw) of the polyolefin contained in the polyolefin microporous membrane is preferably from 500,000 to 5,000,000. When the Mw of the polyolefin is 500,000 or more, sufficient mechanical properties can be imparted to the microporous membrane. When the Mw of the polyolefin is 5,000,000 or less, the microporous membrane is easily molded.

The weight average molecular weight (Mw) of the polyethylene contained in the polyolefin microporous membrane is preferably 800,000 or more, more preferably 1,000,000 or more, still more preferably 1,100,000 or more from the viewpoint of densifying the porous structure of the microporous membrane.

The weight average molecular weight (Mw) of the polyethylene contained in the polyolefin microporous membrane is preferably 2,800,000 or less, more preferably 2,500,000 or less, and still more preferably 2,300,000 or less from the viewpoint of increasing the porosity of the microporous membrane.

The weight average molecular weights of the polyolefin and the polyethylene contained in the polyolefin microporous membrane can be obtained by heating and dissolving the polyolefin microporous membrane in o-dichlorobenzene, and performing measurement by gel permeation chromatography (system: Alliance GPC 2000 manufactured by Waters Corporation, columns: GMH6-HT and GMH6-HTL) under the conditions of a column temperature of 140°C and a flow rate of 1.0 mL/min. Monodisperse polystyrene (manufactured by Tosoh Corporation) is used for calibration of the molecular weight.

An example of an embodiment of the polyolefin microporous membrane includes a microporous membrane containing a polyolefin composition (which means a polyolefin mixture containing two or more types of polyolefins, and is referred to as a polyethylene composition in a case in which the polyolefin contained is only polyethylene in the disclosure). The polyolefin composition has an effect of forming a network structure with fibrillation during stretching and increasing the porosity of the polyolefin microporous membrane.

The polyolefin composition is preferably a polyolefin composition containing ultra-high molecular weight polyethylene having a weight average molecular weight of $9 \times 10^5$ or more in an amount of from 5% by mass to 70% by mass, more preferably from 10% by mass to 60% by mass, and still more preferably from 15% by mass to 50% by mass with respect to the total amount of the polyolefin.

The polyolefin composition is preferably a polyolefin composition obtained by mixing ultra-high molecular weight polyethylene having a weight average molecular weight of $9 \times 10^5$ or more and high-density polyethylene having a weight average molecular weight of from $2 \times 10^5$ to $8 \times 10^5$ and a density of from 920 kg/m$^3$ to 960 kg/m$^3$ at a mass ratio of from 5 : 95 to 70 : 30 (more preferably from 10 : 90 to 60 : 40, still more preferably from 15 : 85 to 50 : 50).

An example of an embodiment of the polyolefin microporous membrane is a polyolefin microporous membrane subjected to a hydrophilization treatment. Examples of the polyolefin microporous membrane subjected to a hydrophilization treatment include a polyolefin microporous membrane having a surface coated with a hydrophilic compound (for example, an ethylene-vinyl alcohol copolymer); a polyolefin microporous membrane obtained by polymerizing a monomer having a hydrophilic group on the surface; and a polyolefin microporous membrane subjected to a plasma treatment or a corona treatment. After the polyolefin microporous membrane and the porous support layer are layered, the entire laminated film may be subjected to a hydrophilization treatment.

[Method for Producing Polyolefin Microporous Membrane]

The polyolefin microporous membrane can be produced, for example, by a production method including the following steps (I) to (IV).

Step (I): a step of preparing a solution containing a polyolefin composition and a volatile solvent having a boiling point of lower than 210°C at atmospheric pressure.

Step (II): a step of obtaining a first gel-like molded material by melt-kneading the solution, extruding the obtained melt-kneaded material from a die, followed by cooling and solidification.

Step (III): a step of obtaining a second gel-like molded material by stretching (primary stretching) the first gel-like molded material in at least one direction and drying the solvent.

Step (IV): a step of stretching (secondary stretching) the second gel-like molded material in at least one direction.

Step (I) is a step of preparing a solution containing a polyolefin composition and a volatile solvent having a boiling point of lower than 210°C at atmospheric pressure. The solution is preferably a thermoreversible sol-gel solution, and a polyolefin composition is solated by heating and dissolving the polyolefin composition in a solvent to prepare a thermoreversible sol-gel solution. The volatile solvent having a boiling point of lower than 210°C at atmospheric pressure is not particularly limited as long as it is a solvent capable of sufficiently dissolving a polyolefin. Examples of the volatile solvent include tetralin (from 206°C to 208°C), ethylene glycol (197.3°C), decalin (decahydronaphthalene, from 187°C to 196°C), toluene (110.6°C), xylene (from 138°C to 144°C), diethyltriamine (107°C), ethylenediamine (116°C), dimethylsulfoxide (189°C), and hexane (69°C), and decalin or xylene is preferable (the temperature in parentheses is the boiling point at atmospheric pressure). The volatile solvents may be used singly, or in combination of two or more kinds thereof.

The polyolefin composition (which means a polyolefin mixture containing two or more types of polyolefins, and is referred to as a polyethylene composition in a case in which the polyolefin contained is only polyethylene in the disclosure) used in Step (I) preferably contains polyethylene, and more preferably is a polyethylene composition.

In the solution prepared in Step (I), the concentration of the polyolefin composition is preferably from 10% by mass to 40% by mass, and more preferably from 15% by mass to 35% by mass from the viewpoint of controlling the porous structure of the polyolefin microporous membrane. When the concentration of the polyolefin composition is 10% by mass or more, the occurrence of cutting can be prevented in the step of forming a polyolefin microporous membrane, and the mechanical strength of the polyolefin microporous membrane is increased to improve the handleability. When the concentration of the polyolefin composition is 40% by mass or less, pores of the polyolefin microporous membrane are easily formed.

Step (II) is a step of obtaining a first gel-like molded material by melt-kneading the solution prepared in Step (I), extruding the obtained melt-kneaded material from a die, followed by cooling and solidification. In Step (II), for example, an extrudate is obtained by extrusion from a die in a temperature range of from the melting point of the polyolefin composition to the melting point + 65°C, and then the extrudate is cooled to obtain a first gel-like molded material. The first gel-like molded material is preferably shaped into a sheet. The cooling may be performed by immersion in water or an organic solvent or may be performed by contacting with a cooled metal roll, and is generally performed by immersion in the volatile solvent used in Step (I).

Step (III) is a step of obtaining a second gel-like molded material by stretching (primary stretching) the first gel-like molded material in at least one direction and drying the solvent. The stretching step in Step (III) is preferably biaxial stretching, and may be sequential biaxial stretching in which longitudinal stretching and transverse stretching are separately performed, or simultaneous biaxial stretching in which longitudinal stretching and transverse stretching are simultaneously performed. The stretch ratio in the primary stretching (the product of the longitudinal stretch ratio by the transverse stretch ratio) is preferably from 1.1 times to 3 times, and more preferably from 1.1 times to 2 times from the viewpoint of controlling the porous structure of the polyolefin microporous membrane. The temperature during stretching in the primary stretching is preferably 75°C or lower. The drying step in Step (III) is performed without any particular restrictions as long as the temperature does not deform the second gel-like molded material, but is preferably performed at 60°C or lower.

The stretching step and the drying step in Step (III) may be performed simultaneously or stepwise. For example, the primary stretching may be performed while pre-drying is performed, and then main drying may be performed, or the primary stretching may be performed between pre-drying and main drying. The primary stretching can be performed even in a state where drying is controlled to allow the solvent to remain in a suitable state.

Step (IV) is a step of stretching (secondary stretching) the second gel-like molded material in at least one direction. The stretching step in Step (IV) is preferably biaxial stretching. The stretching step in Step (IV) may be any of sequential biaxial stretching in which longitudinal stretching and transverse stretching are separately performed; simultaneous biaxial stretching in which longitudinal stretching and transverse stretching are simultaneously performed; a step of performing stretching a plurality of times in the longitudinal direction and then performing stretching in the transverse direction; a step of performing stretching in the longitudinal direction and performing stretching a plurality of times in the transverse direction; and a step of performing sequential biaxial stretching and then further performing stretching once or a plurality of times in the longitudinal direction and/or the transverse direction.

The stretch ratio in the secondary stretching (the product of the longitudinal stretch ratio by the transverse stretch ratio) is preferably from 5 times to 90 times, and more preferably from 10 times to 60 times from the viewpoint of controlling the porous structure of the polyolefin microporous membrane. The stretching temperature in the secondary stretching is preferably from 90°C to 135°C, and more preferably from 90°C to 130°C from the viewpoint of controlling the porous structure of the polyolefin microporous membrane.

A heat fixation treatment may be performed subsequently to Step (IV). The heat fixation temperature is preferably from 110°C to 160°C, more preferably from 120°C to 150°C from the viewpoint of controlling the porous structure of the polyolefin microporous membrane.

After the heat fixation treatment, an extraction treatment of the solvent remaining in the polyolefin microporous membrane and an annealing treatment may be further performed. The extraction treatment of the residual solvent is performed, for example, by immersing the sheet after the heat fixation treatment in a methylene chloride bath to elute the residual solvent in methylene chloride. It is preferable that methylene chloride is removed by drying after the polyolefin microporous membrane immersed in the methylene chloride bath is lifted from the methylene chloride bath. The annealing treatment is performed, for example, by conveying the polyolefin microporous membrane on a roller heated to from 100°C to 140°C after the extraction treatment of the residual solvent.

The Gurley value and the porosity of the polyolefin microporous membrane are adjusted by controlling the respective conditions of Steps (I) to (IV).

[Porous Support Layer]

The porous support layer is a layer for ensuring the strength of the laminated film. The porous support layer is a layer, which has pores or voids inside, and through which a gas or a liquid can pass from one surface to the other surface.

As the porous support layer, a fiber structure of an organic substance or an inorganic substance is suitable. Examples of the fiber structure of an organic substance include a nonwoven fabric or a woven or knitted fabric mainly containing a thermoplastic fiber. Examples of the fiber structure of an inorganic substance include an electrospun fiber membrane containing a glass fiber nonwoven fabric, steel wool, a ceramic fiber, or the like.

The nonwoven fabric, the woven or knitted fabric, or the fiber membrane may be a structure including a laminated structure of two or more layers. The nonwoven fabric, the woven or knitted fabric, or the fiber membrane may be of one type or two or more types in terms of the type of material, the thickness of the fiber, the cross-sectional shape, or the basis weight.

As the porous support layer, a fiber structure mainly containing a thermoplastic fiber is suitable, and in particular, a nonwoven fabric is suitable. In the fiber structure, the thermoplastic fiber is preferably contained in an amount of 70% by mass or more. Examples of the thermoplastic fiber include fibers made of a resin such as a polyester, a polyolefin, a polyamide, a polyesteramide, an acrylic resin, or a polyvinyl alcohol, and mixtures of these fibers. Among these materials, a structure of at least one type of fiber selected from the group consisting of a polyester fiber, a polyolefin fiber, a nylon, an acrylic resin fiber, and a polyvinyl alcohol-based resin fiber is preferable from the viewpoint of a favorable bonding state to the polyolefin microporous membrane and imparting mechanical strength. Among these fiber structures, a polyester fiber structure is more preferable. Examples of the polyester include a polyethylene terephthalate (PET)-based resin, a polybutylene terephthalate (PBT)-based resin, a polytrimethylene terephthalate (PTT)-based resin, a polyethylene naphthalate (PEN)-based resin, a polytrimethylene naphthalate (PTN)-based resin, a polybutylene naphthalate (PBN)-based resin, a polyethylene isophthalate-based resin, and an all aromatic polyester-based resin. Examples of the polyolefin include polypropylene and polyethylene. Examples of the nylon include nylon 6 and nylon 6,6. Examples of the acrylic resin include polyacrylate and polymethyl methacrylate. Examples of the polyvinyl alcohol-based resin include an ethylene-vinyl alcohol copolymer.

In a case in which the porous support layer is an organic fiber structure, the melting point of the resin included in the organic fiber is preferably 100°C or higher, more preferably 150°C or higher, and still more preferably 200°C or higher from the viewpoint of imparting sufficient heat resistance to the porous support layer. The melting point of the resin included in the organic fiber is preferably 300°C or lower, more preferably 280°C or lower, and still more preferably 260°C or lower from the viewpoint of ease of processing from the resin into the organic fiber structure.

The basis weight of the porous support layer is preferably 50 g/m$^2$ or more, more preferably 55 g/m$^2$ or more, and still more preferably 60 g/m$^2$ or more from the viewpoint of high rigidity of the porous support layer.

The basis weight of the porous support layer is preferably 150 g/m$^2$ or less, more preferably 120 g/m$^2$ or less, and still more preferably 100 g/m$^2$ or less from the viewpoint of excellent processability (folding or welding by heat) of the porous support layer.

The bulk density of the porous support layer is preferably 0.20 g/cm$^3$ or more, more preferably 0.25 g/cm$^3$ or more, and still more preferably 0.30 g/cm$^3$ or more from the viewpoint of high rigidity of the porous support layer.

The bulk density of the porous support layer is preferably 0.50 g/cm$^3$ or less, more preferably 0.48 g/cm$^3$ or

less, and still more preferably 0.45 g/cm$^3$ or less from the viewpoint of excellent processability (folding or welding by heat) of the porous support layer.

The thickness of one porous support layer is preferably 100 $\mu$m or more, more preferably 120 $\mu$m or more, and still more preferably 140 $\mu$m or more from the viewpoint of high rigidity of the porous support layer.

The thickness of one porous support layer is preferably 240 $\mu$m or less, more preferably 220 $\mu$m or less, and still more preferably 200 $\mu$m or less from the viewpoint of excellent processability (folding or welding by heat) of the porous support layer.

The thickness of the porous support layer is determined by measurement at 20 points with a film thickness meter and averaging the measurements.

Examples of the method for producing a nonwoven fabric include a production method in which a fiber web is formed and fibers in the fiber web are bonded to obtain a nonwoven fabric. Examples of the method for producing a fiber web include dry methods such as a carding method, an air-laying method, a spunbond method, and a melt-blow method; wet methods such as a wet papermaking method; and an electrostatic spinning method. The wet method is a method in which fibers are dispersed in water to form a uniform paper making slurry, and the paper making slurry is used as a material to obtain a fiber web using a paper machine having at least one papermaking system of a cylinder type, a fourdrinier type, an inclined type, or the like. In the method for producing a nonwoven fabric from a fiber web, fibers are bonded by a fiber bonding method selected from the group consisting of adhesion, fusion, and entanglement. It is also preferable that the nonwoven fabric is subjected to a heating and pressurizing treatment (thermal calendering treatment) by being allowed to pass between a heated metal roll and a heated elastic roll.

As a method for producing a woven or knitted fabric, weaving or knitting are performed from a filament or a spun yarn by a general method, as is performed with a general thermoplastic fiber.

[Bonding Part]

The polyolefin microporous membrane and the porous support layer are bonded to each other by the bonding parts containing a thermoplastic resin. The bonding parts containing a thermoplastic resin are scattered at the boundary surface between the polyolefin microporous membrane and the porous support layer. Since the bonding parts are scattered at the boundary surface between the polyolefin microporous membrane and the porous support layer, the air permeability of the laminated film is ensured. In addition, since the bonding parts are scattered at the boundary surface between the polyolefin microporous membrane and the porous support layer, clogging with biological particles at the boundary surface is prevented from occurring.

In the disclosure, the word "scattered" refers to a state in which the thermoplastic resin included in the bonding part is present in a continuously or discontinuously dispersed manner in a state of not covering the entire surface of the polyolefin microporous membrane between the polyolefin microporous membrane and the porous support layer. For example, the thermoplastic resin is present in the form of a dot, a line, a fiber, a band, a lattice, a net, a three-dimensional network, or the like, and a form in which the resin in such a form is deformed by thermal fusion and/or pressurization is also included.

The bonding part contains a thermoplastic resin and preferably contains only a thermoplastic resin. That is, the bonding part is preferably composed of a thermoplastic resin.

The melting point of the thermoplastic resin contained in the bonding part is preferably lower than the melting point of the polyolefin contained in the polyolefin microporous membrane. In a case in which the porous support layer contains a resin, the melting point of the thermoplastic resin contained in the bonding part is preferably lower than the melting point of the resin contained in the porous support layer.

In a case in which the polyolefin microporous membrane contains two or more types of polyolefins, the melting point of the thermoplastic resin contained in the bonding part is preferably lower than the melting points of all the polyolefins contained in the polyolefin microporous membrane.

In a case in which the bonding part contains two or more types of thermoplastic resins, the melting points of all the thermoplastic resins are preferably lower than the melting point of the polyolefin contained in the polyolefin microporous membrane.

In a case in which the polyolefin microporous membrane contains two or more types of polyolefins and the bonding part contains two or more types of thermoplastic resins, the melting points of all the thermoplastic resins contained in the bonding part are preferably lower than the melting points of all the polyolefins contained in the polyolefin microporous membrane.

The melting point of the thermoplastic resin contained in the bonding part is preferably 50°C or higher, more preferably 60°C or higher, and still more preferably 70°C or higher from the viewpoint of preventing deformation of the bonding part or elution of a component contained in the bonding part due to the temperature of a gas or a liquid to be circulated.

The melting point of the thermoplastic resin contained in the bonding part is preferably 130°C or lower, more preferably 125°C or lower, and still more preferably 120°C or lower from the viewpoint of reducing the temperature of heat applied for bonding the polyolefin microporous membrane and the porous support layer to prevent deformation of the polyolefin microporous membrane and the porous support layer.

The melting point of the thermoplastic resin is the "melting peak temperature" of a differential scanning calorimetry (DSC) curve obtained according to JIS K 7121: 1987 "Testing Methods for Transition Temperatures of Plastics".

Examples of the thermoplastic resin contained in the bonding part include resins such as a polyolefin, a polyester, an acrylic resin, and a polyvinyl alcohol. Among these materials, a polyolefin is preferable from the viewpoint of forming a favorable bonding state to at least the polyolefin microporous membrane. Examples of the polyolefin include polyethylene, polypropylene, polybutylene, polymethylpentene, and a copolymer of polypropylene and polyethylene. The thermoplastic resin contained in the bonding part is preferably the same type of polyolefin as the polyolefin contained in the polyolefin microporous membrane. For example, in a case in which the polyolefin microporous membrane is a polyethylene microporous membrane, the thermoplastic resin contained in the bonding part is preferably polyethylene.

When the boundary surface between the polyolefin microporous membrane and the porous support layer is viewed in a plan view, the bonding parts are scattered in the form of, for example, a dot, a line, a lattice, or a net. The bonding part may or need not be visually observable.

When the bonding part is in the form of a dot, the number of bonding parts is preferably 3,000 to 15,000 per 10 cm square.

When the boundary surface between the polyolefin microporous membrane and the porous support layer is viewed in a plan view, the total area of the bonding parts is preferably from 5% to 80% with respect to the area of the boundary surface.

[Method for Producing Laminated Film]

The laminated film of the disclosure is produced, for example, by a production method including the following steps (a) to (c).

Step (a): a step of disposing a thermoplastic resin on one surface of a first layer included in the laminated film in a scattered manner.

Step (b): a step of forming a laminated body by superimposing a second layer included in the laminated film on the thermoplastic resin scattered on the surface of the first layer.

Step (c): a step of bonding the first layer and the second layer to each other by allowing the laminated body to pass through a heating device to dissolve the thermoplastic resin.

The first layer is either a polyolefin microporous membrane or a porous support layer. In a case in which the first layer is a polyolefin microporous membrane, the second layer is a porous support layer, and in a case in which the first layer is a porous support layer, the second layer is a polyolefin microporous membrane.

Step (a) and Step (b) are performed once or a plurality of times according to the number of layers of the laminated film. For example, in a case in which the laminated film includes three layers, the second layer in the first Step (b) is the first layer in the second Step (a).

The thermoplastic resin used in Step (a) functions as an adhesive for bonding the first layer and the second layer. The thermoplastic resin used in Step (a) is preferably in the form of a particle, a line, or a fiber.

The amount of the thermoplastic resin used is preferably from 1 g/m$^2$ to 20 g/m$^2$ with respect to the surface of the first layer.

The coverage of the thermoplastic resin that covers the surface of the first layer is preferably from 5% to 80%.

The heating device in Step (c) is, for example, a heating and pressurizing roll; a heating roll and a pressurizing roll; or a heating furnace including a pressurizing roll.

The temperature of the heating device is preferably higher than the melting point of the thermoplastic resin functioning as an adhesive - 10°C, and lower than the melting point of the polyolefin contained in the polyolefin microporous membrane + 10°C. In a case in which the porous support layer contains a resin, the temperature of the heating device is preferably lower than the melting point of the resin contained in the porous support layer. The temperature of the heating device is preferably from 50°C to 140°C, more preferably from 60°C to 135°C, and still more preferably from 70°C to 130°C. The time for applying heat by the heating device is set to a time for the thermoplastic resin functioning as an adhesive to melt sufficiently.

In a case in which the heating device includes a roll member, a pressure applied by the roll member is set within a range in which the porous structure of the polyolefin microporous membrane is not blocked.

Examples

Hereinafter, the laminated film of the disclosure will be described more specifically with reference to examples. Materials, amounts used, proportions, treatment procedures, and the like shown in the following examples can be changed as appropriate unless the gist of the disclosure is exceeded. Therefore, the scope of the laminated film of the disclosure should not be restrictively interpreted by the following specific examples.

In the following description, synthesis, treatment, production, and the like were performed at room temperature (25°C ± 3°C) unless otherwise specified.

<Measurement Method and Evaluation Method>

Measurement methods and evaluation methods applied to examples and comparative examples are as follows.

[Weight Average Molecular Weight of Polyolefin]

The weight average molecular weight of the polyolefin contained in the polyolefin microporous membrane was measured by gel permeation chromatography (system: Alliance GPC 2000 manufactured by Waters Corporation, columns: GMH6-HT and GMH6-HTL) under the conditions of a column temperature of 140°C and a flow rate of 1.0 mL/min after the polyolefin microporous membrane was heated and dissolved in o-dichlorobenzene. Monodisperse polystyrene (manufactured by Tosoh Corporation) was used for calibration of the molecular weight.

[Gurley Value of Laminated Film]

Measurement was performed using a Gurley type densometer (Toyo Seiki Seisakusho, Ltd., model number: G-B2C) according to JIS P 8117: 2009.

[Average Thickness of Polyolefin Microporous Membrane]

The average thickness was determined by measurement at 20 points with a contact-type film thickness meter (Mitutoyo Corporation), and averaging the measurements. As the contact terminal, a cylindrical terminal having a bottom surface with a diameter of 0.5 cm was used. The measurement pressure was set to 0.1 N.

[Porosity of Polyolefin Microporous Membrane]

The porosity was determined from the following formula. Here, regarding a component material 1, a component material 2, a component material 3, ..., and a component material n of the polyolefin microporous membrane, the mass of each component material is denoted by $W_1$, $W_2$, $W_3$, ..., and $W_n$ (g/cm$^2$), the true density of each component material is denoted by $d_1$, $d_2$, $d_3$, ..., and $d_n$ (g/cm$^3$), and the average thickness of the polyolefin microporous membrane is denoted by t (cm).

$$\varepsilon = \left( 1 - \frac{\sum_{i=1}^{n} \frac{Wi}{di}}{t} \right) \times 100$$

[Basis Weight of Porous Support Layer]

The basis weight was determined by measuring the mass of a sample obtained by cutting the porous support layer before being layered into a 10 cm × 10 cm square, and dividing the mass of the sample by the area (100 cm$^2$).

[Bulk Density of Porous Support Layer]

The bulk density was determined by measuring the mass and the thickness of a sample obtained by cutting the porous support layer before being layered into a 10 cm × 10 cm square, and dividing the mass of the sample by the thickness and the area (100 cm$^2$). The thickness was determined by measurement at 20 points with a Digimatic Micrometer (Mitutoyo Corporation, model number: MDC-25MJ) and averaging the measurements.

[Initial Pressure Loss]

The laminated film was cut out and placed in a holder having an effective opening diameter of 40 mm with the surface on which the polyolefin microporous membrane is exposed facing upstream. Air was allowed to pass through the holder at a flow rate of 5.3 cm/sec, and a pressure difference (Pa) between the upstream and the downstream of the laminated film was measured with a micro differential pressure gauge. The measured pressure difference (Pa) was categorized as follows. The practically allowable range is a pressure difference of less than 40 kPa.

A: less than 10 kPa
B: 10 kPa or more and less than 40 kPa
C: 40 kPa or more

[Bacteria Separation Performance]

The following tools were prepared.

· Oilless air compressor, model number: ACP-10A, TAKAGI CO., LTD. Hereinafter, this is referred to as an "air compressor".
· Baby tank for lab tests, model number: BT-700S, ADVANTEC. Hereinafter, this is referred to as a "pressure tank".
· Stainless steel line holder, model number: KS-47, ADVANTEC. Hereinafter, this is referred to as a "holder".
· Membrane filter having a pore size of 0.22 $\mu$m, model number: A020B025A, ADVANTEC. Hereinafter, this is referred to as a "membrane filter".

The laminated film was cut into a circle having a diameter of 47 mm, and this was used as a sample. The sample was immersed in ethanol, and then the sample wetted with ethanol was placed inside the holder with the surface on which the polyolefin microporous membrane is exposed facing upstream.
The following (1) to (5) were performed to determine a logarithmic reduction value (LRV).

(1) Preparation of Test Bacterial Solution

Test bacteria were seeded in TSA medium and cultured at a temperature of 30°C for 24 hours. A grown colony was suspended in 10 mL of TSB medium and cultured at a temperature of 30°C for 24 hours. To 1,000 mL of a salted lactose broth medium, 2 mL of this culture solution was added dropwise, and cultured at a temperature of 30°C for 24 hours. This culture solution was diluted 10 times with physiological saline and mixed well to prepare a test bacterial solution.

(2) Measurement of Cell Count in Test Bacterial Solution

The test bacterial solution was serially diluted up to 10 times with physiological saline. On SA medium, 0.1 mL of each of the test bacterial solution and the diluted solutions was smeared, and cultured at a temperature of 30°C for 48 hours, and the number of grown colonies was counted. From the number of colonies measured, the cell count per 500 mL of the test bacterial solution was determined.

(3) Bacteria Separation Operation

An air compressor was connected to a pressure tank in which about 550 mL of the test bacterial solution was placed, and a valve was closed. Compressed air was fed from the air compressor, and the inside of the pressure tank was pressurized to 0.21 MPa. The valve was opened, and the entire amount of the test bacterial solution was allowed to pass through the holder in which the sample was placed and collected in a water collection container. After the entire amount of the test bacterial solution was allowed to pass through the holder, pressurization by the air compressor was stopped, and the inside of the pressure tank was returned to atmospheric pressure. Hereinafter, the liquid collected in the water collection container is referred to as a "treated liquid".

(4) Measurement of Cell Count in Treated Liquid

A 50 mL portion and a 450 mL portion of the treated liquid were each filtered through a membrane filter. Since the test bacteria have a size that is difficult to pass through the membrane filter, most of the test bacteria remain on the membrane filter. The membrane filter after the treated liquid was filtered was attached to SA medium, and cultured at a temperature of 30°C for 3 days, and then the number of grown colonies was counted. From the number of colonies measured, the cell count per 500 mL of the test bacterial solution was determined.

(5) Calculation of LRV

The LRV was calculated from the following formula.

LRV = log 10(cell count per 500 mL of test bacterial solution/cell count per 500 mL of treated solution)
A LRV of 6 or more was determined to be excellent in bacteria separation performance.

<Production of Laminated Film>

[Example 1]

-Production of Polyethylene Microporous Membrane-

A polyethylene composition in which 12.5 parts by mass of ultra-high molecular weight polyethylene (hereinafter referred to as "UHMWPE") having a weight average molecular weight of 4,600,000 was mixed with 12.5 parts by mass of high-density polyethylene (hereinafter referred to as "HDPE") having a weight average molecular weight of 560,000 and a density of 950 kg/m$^3$ was prepared. The polyethylene composition and decalin were mixed so that the polymer concentration was 25% by mass to prepare a polyethylene solution.
The polyethylene solution was extruded from a die at a temperature of 152°C into a sheet, and then the extrudate was cooled in a water bath at a water temperature of 20°C to obtain a first gel-like sheet.
The first gel-like sheet was pre-dried in a temperature atmosphere of 70°C for 10 minutes, then subjected to primary stretching at 1.2 times in the MD direction, and then subjected to main drying in a temperature atmosphere of 57°C for 5 minutes to obtain a second gel-like sheet (base tape) (the residual amount of the solvent in the second gel-like sheet was set to less than 1% by mass). Subsequently, as secondary stretching, the second gel-like sheet (base tape) was stretched at a ratio of 3 times in the MD direction at a temperature of 90°C, then stretched at a ratio of 10 times in the TD direction at a temperature of 115°C, and immediately thereafter subjected to a heat treatment (heat fixation) at 135°C.
Decalin in the sheet was extracted while the sheet after heat fixation was immersed in a methylene chloride bath divided into two tanks successively for 30 seconds each. After the sheet was taken out from the methylene chloride bath, methylene chloride was removed by drying in a temperature atmosphere of 40°C. A polyethylene microporous membrane was obtained in this manner.
-Layering of Polyethylene Microporous Membrane and Polyester Nonwoven Fabric-As a porous support layer, a polyester nonwoven fabric having the basis weight and the bulk density shown in Table 1 was prepared. A polyethylene powder having a melting point of 105°C was sprayed in an amount of 5 g per square meter on one surface of the polyester nonwoven fabric, and the polyethylene microporous membrane was superimposed thereon, and the polyethylene microporous membrane and the polyester nonwoven fabric were bonded to each other by applying a temperature of 100°C using a heating and pressurizing roll.

[Examples 2 to 8 and Comparative Examples 1 and 2]

-Production of Polyolefin Microporous Membrane-

Polyethylene microporous membranes having the physical properties shown in Table 1 were produced by changing the mixing ratio of UHMWPE and HDPE so that the weight average molecular weight of the polyethylene measured by the above-mentioned method was the value shown in Table 1, and controlling the respective conditions of the production steps. In Example 7, a polypropylene microporous membrane was produced using polypropylene in place of the polyethylene.

-Layering of Polyolefin Microporous Membrane and Nonwoven Fabric

As the specification shown in Table 1, the polyethylene microporous membrane or the polypropylene microporous membrane was bonded to one surface of the polyester nonwoven fabric or the polypropylene nonwoven fabric using a polyethylene powder or a polyethylene web. The melting points of the polyethylene powder and the polyethylene web are 105°C. As the polyethylene web, a web-like (nonwoven fabric-like) polyethylene having a basis weight of 12 g/m$^2$ was used.

[Table 1]

| | Polyolefin microporous membrane | | | | | Porous support layer | | | Laminated film | | | | |
| | Resin | | Gurley value | Porosity | Average thickness | Type | Basis weight | Bulk density | Layered form | Thermoplastic resin for bonding | Gurley value | Initial pressure loss | Bacteria separation performance |
| | Type | Mw | | | | | | | | | | | |
| | --- | $\times 10^4$ | sec/100 mL | % | $\mu$m | --- | g/m$^2$ | g/cm$^3$ | --- | --- | sec/100 mL | kPa | LRV |
| Example 1 | PE | 216 | 8 | 83 | 41 | PET nonwoven fabric | 75 | 0.44 | One surface | PE powder | 12 | A | 8.7 |
| Example 2 | PE | 102 | 3 | 85 | 93 | PET nonwoven fabric | 75 | 0.44 | One surface | PE powder | 8 | A | 8.5 |
| Example 3 | PE | 184 | 9 | 83 | 17 | PET nonwoven fabric | 75 | 0.44 | One surface | PE powder | 17 | A | > 8.9 |
| Example 4 | PE | 118 | 61 | 61 | 12 | PET nonwoven fabric | 75 | 0.44 | One surface | PE powder | 90 | B | > 8.9 |
| Example 5 | PE | 216 | 8 | 83 | 41 | PET nonwoven fabric | 75 | 0.44 | One surface | PE web | 20 | A | > 8.9 |
| Example 6 | PE | 216 | 8 | 83 | 41 | PET nonwoven fabric | 70 | 0.35 | One surface | PE powder | 16 | A | > 8.9 |
| Example 7 | PP | --- | 12 | 84 | 52 | PET nonwoven fabric | 75 | 0.44 | One surface | PE powder | 15 | A | > 8.9 |
| Example 8 | PE | 216 | 8 | 83 | 41 | PP nonwoven fabric | 80 | 0.41 | One surface | PE powder | 20 | A | > 8.9 |
| Comparative Example 1 | PE | 69 | 0.6 | 91 | 139 | PET nonwoven fabric | 75 | 0.44 | One surface | PE powder | 4 | A | < 3.7 |

(continued)

| | Polyolefin microporous membrane | | | | | Porous support layer | | | Laminated film | | | | |
| | Resin | | Gurley value | Porosity | Average thickness | Type | Basis weight | Bulk density | Layered form | Thermoplastic resin for bonding | Gurley value | Initial pressure loss | Bacteria separation performance |
| | Type | Mw | | | | | | | | | | | |
| | --- | $\times 10^4$ | sec/100 mL | % | $\mu$m | --- | g/m$^2$ | g/cm$^3$ | --- | --- | sec/100 mL | kPa | LRV |
| Comparative Example 2 | PE | 282 | 103 | 59 | 11 | PET nonwoven fabric | 75 | 0.44 | One surface | PE powder | 132 | C | > 8.9 |

Abbreviations in Table 1 have the following meanings.

· Mw: Weight average molecular weight
· LRV: Logarithmic Reduction Value
· PE: Polyethylene
· PP: Polypropylene
· PET: Polyethylene terephthalate

The disclosure of Japanese Patent Application No. 2021-130802 filed on August 10, 2021 is incorporated herein by reference in its entirety.

All documents, patent applications, and technical standards described in this description are incorporated herein by reference to the same extent as if each individual document, patent application, and technical standard were specifically and individually indicated to be incorporated by reference.

**Claims**

1. A laminated film, comprising:

   a microporous membrane containing a polyolefin; and
   a porous support layer, wherein:

   the microporous membrane and the porous support layer are bonded to each other by bonding parts that contain a thermoplastic resin and are scattered, and
   the laminated film has a Gurley value of from 5 sec/100 mL to 100 sec/100 mL.

2. The laminated film according to claim 1, wherein the microporous membrane contains polyethylene.

3. The laminated film according to claim 2, wherein the polyethylene contained in the microporous membrane has a weight average molecular weight of from 800,000 to 2,800,000.

4. The laminated film according to any one of claims 1 to 3, wherein the microporous membrane has a Gurley value of from 2 sec/100 mL to 100 sec/100 mL.

5. The laminated film according to any one of claims 1 to 4, wherein the microporous membrane has a porosity of from 80% to 90%.

6. The laminated film according to any one of claims 1 to 5, wherein the microporous membrane has an average thickness of from 10 $\mu$m to 110 $\mu$m.

7. The laminated film according to any one of claims 1 to 6, wherein the porous support layer is a polyester fiber structure.

8. The laminated film according to any one of claims 1 to 7, wherein the porous support layer has a basis weight of from 50 g/m$^2$ to 150 g/m$^2$.

9. The laminated film according to any one of claims 1 to 8, wherein the porous support layer has a bulk density of from 0.20 g/cm$^3$ to 0.50 g/cm$^3$.

10. The laminated film according to any one of claims 1 to 9, wherein the laminated film is used for separating bacteria.

FIG.1

FIG.2

## INTERNATIONAL SEARCH REPORT

International application No.

**PCT/JP2022/030003**

### A. CLASSIFICATION OF SUBJECT MATTER

***B32B 5/32***(2006.01)i; ***B01D 69/00***(2006.01)i; ***B01D 69/02***(2006.01)i; ***B01D 69/10***(2006.01)i; ***B01D 69/12***(2006.01)i; ***B01D 71/14***(2006.01)i; ***B01D 71/26***(2006.01)i; ***B32B 27/32***(2006.01)i; ***C12M 1/00***(2006.01)i; ***C12N 1/02***(2006.01)i
FI: B32B5/32; B32B27/32 Z; B01D69/10; B01D69/12; B01D71/26; B01D69/02; B01D69/00; B01D71/14; C12M1/00 Z; C12N1/02

According to International Patent Classification (IPC) or to both national classification and IPC

### B. FIELDS SEARCHED

Minimum documentation searched (classification system followed by classification symbols)

B32B5/32; B01D69/00; B01D69/02; B01D69/10; B01D69/12; B01D71/14; B01D71/26; B32B27/32; C12M1/00; C12N1/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 05-138786 A (UNITIKA LTD.) 08 June 1993 (1993-06-08) claims, examples | 1-7, 9, 10 |
| Y | | 8 |
| Y | JP 10-066847 A (TEIJIN LTD.) 10 March 1998 (1998-03-10) claims, paragraph [0019] | 8 |
| A | JP 07-047633 A (MITSUBISHI CHEMICAL CORP.) 21 February 1995 (1995-02-21) claims, examples, entire text | 1-10 |
| A | JP 2017-522210 A (E.I. DU PONT DE NEMOURS AND CO.) 10 August 2017 (2017-08-10) claims, examples, entire text | 1-10 |
| A | JP 2014-061505 A (TEIJIN LTD.) 10 April 2014 (2014-04-10) claims, examples, entire text | 1-10 |
| A | WO 2020/028328 A1 (W. L. GORE & ASSOCIATES, INC.) 06 February 2020 (2020-02-06) claims, examples, entire text | 1-10 |

☐ Further documents are listed in the continuation of Box C.    ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **27 September 2022** | **11 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/030003**

| Patent document cited in search report | | | Publication date (day/month/year) | Patent family member(s) | | | Publication date (day/month/year) |
|---|---|---|---|---|---|---|---|
| JP | 05-138786 | A | 08 June 1993 | (Family: none) | | | |
| JP | 10-066847 | A | 10 March 1998 | WO | 1998/001219 | A1 | |
| | | | | claims, p. 8, lines 2-8 | | | |
| | | | | EP | 0862491 | A1 | |
| | | | | CN | 1197409 | A | |
| | | | | KR | 10-1999-0044425 | A | |
| JP | 07-047633 | A | 21 February 1995 | (Family: none) | | | |
| JP | 2017-522210 | A | 10 August 2017 | US | 2016/0016385 | A1 | |
| | | | | claims, examples, entire text | | | |
| | | | | WO | 2016/011154 | A1 | |
| | | | | EP | 3169514 | A1 | |
| | | | | CN | 106488842 | A | |
| | | | | CN | 113386433 | A | |
| JP | 2014-061505 | A | 10 April 2014 | (Family: none) | | | |
| WO | 2020/028328 | A1 | 06 February 2020 | JP | 2021-532287 | A | |
| | | | | claims, examples, entire text | | | |
| | | | | US | 2021/0317276 | A1 | |
| | | | | EP | 3830172 | A1 | |
| | | | | CN | 112543785 | A | |
| | | | | KR | 10-2021-0036960 | A | |
| | | | | CA | 3105755 | A | |

Form PCT/ISA/210 (patent family annex) (January 2015)

**EP 4 385 724 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011512252 A
- JP 2015163465 A
- JP H11179120 A
- JP 2008114530 A
- JP 2021130802 A